(19) Europäisches Patentamt
European Patent Office
Office européen des brevets

(11) Numéro de publication : **0 301 969 B1**

(12) # FASCICULE DE BREVET EUROPEEN

(45) Date de publication du fascicule du brevet : **21.08.91 Bulletin 91/34**

(51) Int. Cl.$^5$ : **A61K 31/47**, A61K 9/22, A61K 9/16

(21) Numéro de dépôt : **88401965.4**

(22) Date de dépôt : **28.07.88**

(54) **Microparticules comportant un polymère biodégradable contrôlant la libération d'un principe actif antimalarique, compositions pharmaceutiques en comprenant et procédé de préparation.**

(30) Priorité : **30.07.87 FR 8710802**

(43) Date de publication de la demande : **01.02.89 Bulletin 89/05**

(45) Mention de la délivrance du brevet : **21.08.91 Bulletin 91/34**

(84) Etats contractants désignés : **AT BE CH DE ES FR GB GR IT LI LU NL SE**

(56) Documents cités :
**EP-A- 0 134 318**
**FR-A- 2 070 153**
**CHEMICAL ABSTRACTS, vol. 85, 1976, page 315, résumé no. 182354d, Columbus, Ohio, US; D.L. WISE et al.: "Sustained release of an antimalarial drug using a copolymer of glycolic/lactic acid", & LIFE SCI. 1976, 19(6), 867-73**
**JOURNAL OF PHARMACEUTICAL SCIENCES, vol. 76, no. 2, février 1987, pages 134-140, American Pharmaceutical Association, Washington, US; T. LAAKSO et al.: "Biodegradable microspheres VI: Lysosomal release of covalently bound antiparasitic drugs from starch microparticles"**
**JOURNAL OF PHARMACEUTICAL SCIENCES, vol. 73, no. 12, décembre 1984, pages 1721-1724, American Pharmaceutical Association, Washington, US; S. BENITA et al.: "Characterization of drug-loaded poly(d,l-lactide) microspheres"**

(73) Titulaire : **LA REGION WALLONNE**
**7 Avenue du Prince de Liège**
**B-5100 Namur (BE)**

(72) Inventeur : **Bontemps José**
**Rue de Fêchereux 15**
**B-4430 Xhendremael (Ans) (BE)**
Inventeur : **Pirson Philippe**
**25 Avenue de Burbure**
**B-1970 Wezembeek-Oppem (BE)**
Inventeur : **Falmagne Jean-Bernard**
**19 Montagne d'Aisemont**
**B-1300 Wavre (BE)**
Inventeur : **Jerome Robert**
**6, rue des Sorbiers**
**B-4040 Tilff (BE)**
Inventeur : **Teyssié Philippe**
**Avenue Bois Impérial de Rognac 85**
**B-4121 Neuville en Condroz ( Neupré ) (BE)**
Inventeur : **Delattre Luc**
**Rue Henry Gérard 9**
**B-4480 Oupeye (BE)**
Inventeur : **Evrard Brigitte**
**Grand Route 6**
**B-4248 Verlaine (BE)**

(74) Mandataire : **Ahner, Francis et al**
**CABINET REGIMBEAU, 26, avenue Kléber**
**F-75116 Paris (FR)**

**EP 0 301 969 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La chimiothérapie moderne vise à réduire la toxicité, à prolonger la durée d'action et à augmenter la sélectivité de nombreux médicaments, c'est-à-dire d'une manière générale à augmenter l'index thérapeutique des principes actifs. Pour atteindre ces objectifs, on a recours notamment à l'utilisation de matériaux polymères contrôlant la libération du principe actif.

Il existe à l'heure actuelle des matériaux polymères susceptibles d'assurer le contrôle de la vitesse de libération et de la durée d'action de médicaments. Ces systèmes ont été initialement développés pour la contraception avec les hormones stéroïdiennes. Ils sont, à ce jour, utilisés pour une large gamme de principes actifs, tant micro- que macromoléculaires.

Plus précisément, la présente invention concerne l'incorporation d'agents antimalariques dans des biomatériaux polymères par microencapsulation.

La microencapsulation regroupe l'ensemble des techniques permettant l'obtention de particules individualisées dont la taille s'échelonne entre 1 et 1250 µm.

Les microparticules ainsi obtenues peuvent être divisées en deux groupes :

— les microcapsules, particules sphériques constituées d'une enveloppe solide contenant un liquide, un solide ou une substance pâteuse. Chaque microcapsule constitue un système réservoir proprement dit ;

— les microsphères, particules également sphérique mais constituées d'un réseau continu de matériau support dans lequel est dispersée la substance à encapsuler, à l'état moléculaire ou particulaire. Cette structure, homogène ou hétérogène selon les cas, constitue un système matriciel.

Des agents antimalariques concernés par la présente invention ont été décrits dans les demandes de brevet FR 8704079 (2612778) et BE 200041 (BE882541). Il s'agit de la primaquine et ses dérivés tels que les dérivés acide(s) aminé(s)-primaquine, les conjugués macromoléculaires ciblés, tels que la primaquine liée à l'albumine galactosylée par l'intermédiaire d'un bras tétrapeptidique qui permet la libération d'une forme active de la primaquine au niveau des lysosomes des cellules ciblées. Ces agents antimalariques visent à combattre les formes hépatocytaires de la malaria.

FR 2070153 décrit des particules obtenues par fusion de polymères libérant des stéroïdes dans une période de temps donné.

L'article Life Sciences, volume 19, page 867-874, 1976 décrit des particules composées d'un copolymère d'acide lactique et d'acide glycolique, de taille inférieure à 125 µ et contenant des dérivés de quinazoline antimalarique libérés sur une période de 14 semaines.

Une difficulté des inventions dans le domaine qui nous intéresse est la nécessité de déterminer les polymères présentant des propriétés définies en fonction du type de principe actif à incorporer. Il convient également de déterminer pour un type de drogue donnée la vitesse de libération et le taux de charge assurant une couverture thérapeutique optimum pour une période choisie.

De même, la technique d'incorporation dépend du couple polymère-médicament, notamment de l'existence d'un solvant commun des partenaires, de l'existence de propriétés thermiques de ceux-ci ou de leur miscibilité. En outre, cette incorporation ne doit pas altérer les propriétés du principe actif.

Il existe une grande variété de monomères que l'on peut polymériser par différentes méthodes pour former des chaînes macromoléculaires. En jouant sur la nature des monomères et leur mode d'enchaînement, la chimie macromoléculaire peut donc générer une large gamme de matériaux. Dans un contexte biomédical, il est crucial que les polymères utilisés soient biocompatibles hémocompatibles et/ou biodégradables, le choix des polymères étant également en partie dicté par la voie d'administration.

Etant donné que la malaria se localise dans les pays en voie de développement où l'encadrement, tant médical que paramédical, est extrêmement limité, il convient d'envisager la mise au point de forme galénique injectable dont l'administration est la plus simple.

Un des buts de la présente invention est donc la réalisation de formes microparticulaires injectables permettant de moduler les cinétiques de libération au moyen de matériaux polymères pour une série de molécules actives contre la malaria.

Les médicaments absorbés par voie orale (comprimés, tablettes ou microparticules dans une gélule) peuvent être enrobés par un polymère filmogène non biodégradable, puisqu'il s'éliminera par le tractus après s'être délité au niveau de l'estomac ou à l'entrée de l'intestin. En revanche, si l'on envisage des formes injectables, les polymères doivent présenter des propriétés particulières, notamment de biodégradabilité.

La présente invention a donc pour objet des microparticules contenant un principe actif contre la malaria, tel que la primaquine, un de ses dérivés ou leurs conjugués avec un vecteur hépatotrope ou leurs sels pharmaceutiquement acceptables, et un polymère biocompatible et biodégradable contrôlant la cinétique de libération du principe actif.

Des dérivés de primaquine incorporés selon l'invention sont représentés schématiquement par la formule

PQ-X, dans laquelle

— PQ représente la primaquine,

— X représente un amino-acide ou un peptide de 2 à 4 amino-acides,

— la liaison PQ-X étant une liaison covalente peptidique entre le groupement amine libre de PQ et le groupement carboxylique de X.

Les produits de base PQ ainsi que les dérivés tels que PQ-X peuvent se présenter sous forme de leurs sels d'addition avec des acides.

La préparation de ces dérivés PQ-X a été décrite par la demanderesse notamment dans la demande de brevet FR 8704079.

Selon un mode de réalisation particulier du procédé, on part d'un sel par exemple du diphosphate de primaquine et de l'acide aminé ou peptide dont la fonction aminée est protégée par exemple par un groupe terbutyloxycarbonyle et on effectue les étapes suivantes :

a) on fait réagir de la N-hydroxysuccinimide ou tout autre groupe activant la fonction acide de l'acide aminé ou du peptide, sur le dérivé protégé de l'acide aminé ou peptide, par exemple sur le dérivé N-terbutyloxycarbonyle de l'acide aminé ou peptide,

b) on fait réagir la primaquine, libérée à partir de son sel par exemple par une solution d'ammoniaque, on la fait réagir donc avec par exemple l'ester N-hydroxysuccinimide du dérivé protégé de l'acide aminé ou peptide,

c) le produit brut ainsi obtenu est purifié par chromatographie sur silicagel,

d) le groupement protecteur, tel que le terbutyloxycarbonyle du composé obtenu est ensuite clivé en présence d'acide, par exemple l'acide trifluoroacétique pour donner le dérivé PQ-X sous forme de sel, par exemple trifluoroacétate.

La primaquine agit comme un oxydant et déstabilise la membrane des globules rouges provoquant une hémolyse qui est à l'origine de sa toxicité principale.

L'adjonction d'un amino-acide ou d'un peptide réduit la pénétration de la primaquine dans les globules rouges en raison de l'encombrement stérique.

En outre, l'activité de ces nouveaux dérivés est apparue beaucoup plus importante.

D'un intérêt tout particulier sont les dérivés PQ-X dans lesquels X représente un amino-acide ou un dipeptide.

Parmi ces amino-acides, on peut citer notamment la L-leucine, la D-leucine, la L-alanine, la D-alanine, la L-isoleucine, la L-phénylalanine, l'acide L-glutamique, la L-lysine, la L-tyrosine et la L-glutamine.

Parmi les peptides particulièrement intéressants, on peut citer des dipeptides L-alanyl-L-leucyl, L-leucyl-L-alanyl, mais aussi les tétrapeptides $(L\text{-alanyl-}L\text{-leucyl})_2$, $(L\text{-alanyl})_3$ L-leucyl, L-alanyl-L-leucyl-glycyl-L-leucyl, glycyl-L-leucyl-glycyl-L-leucyl.

Les conjugués avec un vecteur hépatotrope peuvent être par exemple des conjugués macromoléculaires ciblés de primaquine liée à l'albumine galactosylée par l'intermédiaire d'un bras tétrapeptidique décrits dans BE 200041, mais avantageusement le vecteur hépatotrope aura un faible poids moléculaire et sera synthétique.

Plus particulièrement, les microparticules selon l'invention sont des microsphères constituées d'une matrice de polymères au sein de laquelle est distribué le principe actif antimalarique.

Dans un mode de réalisation particulier de l'invention, le polymère est du polylactide.

Parfaitement atoxiques, car métabolisés en acides lactiques, les polymères polylactides se caractérisent par une biodégradabilité dont la cinétique de dégradation convient particulièrement pour contrôler la libération de la primaquine et ses dérivés. En revanche, les polyglycolides par exemple bien qu'également biodégradables, présentent une vitesse de biodégradation trop rapide pour ce type de principe actif et les polycaprolactones présentent une vitesse de dégradation trop lente pour le type d'application envisagée par la présente invention.

Le polylactide est de préférence sous forme (DL).

Le poids moléculaire du polymère est compris entre 50.000 et 100.000.

Le rapport en poids principe actif/polymère est compris entre 15 et 25%.

Dans un mode préférentiel de réalisation de l'invention, les microparticules sont formulées dans un soluté injectable. Pour ce faire, les microparticules doivent avoir une dimension comprise entre 160 et 200 µm.

La présente invention a également pour objet des compositions pharmaceutiques contenant de telles microparticules, notamment des compositions pharmaceutiques injectables.

Un autre objet de la présente invention est un procédé de préparation de ces microparticules selon lequel:

- un polymère est dissous dans un solvant volatil,
- à cette solution on ajoute le principe actif et éventuellement une substance réglant la taille des microparticules,
- le solvant est évaporé sous agitation,

• à l'issue de l'évaporation, des microparticules sont récupérées par centrifugation et filtration.

La primaquine et ses dérivés étant hydrosolubles, dans un mode particulier de réalisation du procédé selon l'invention, on incorpore la primaquine ou un de ses sels pharmaceutiquement acceptables dans du polylactide, le solvant étant l'acétone, le milieu non miscible dispersant étant une huile minérale, telle que la paraffine, la substance réglant la taille des particules étant un agent tensio-actif non ionique.

La forme (DL) du polylactide qui est la plus soluble dans l'acétone convient de manière très appropriée pour ce type de procédé.

Le rapport en poids polymère/solvant est avantageusement de 10 à 30%, de préférence de 15 à 20%.

De préférence, l'émulsification se fait sous agitation violente. On arrive ainsi à obtenir des microparticules de taille inférieure à 200 µm telle que préférée pour rendre ces microparticules injectables.

On peut citer comme agents tensio-actifs non ioniques particulièrement appropriés les esters de sorbitane notamment le monooléate de sorbitane, dans une concentration comprise entre 1 et 10% par rapport au poids du solvant.

Ces caractéristiques sont également importantes en vue d'obtenir des microparticules de forme et de taille appropriées.

D' autres caractéristiques et avantages de la présente invention apparaîtront à la lumière de la description qui va suivre.

Cette description fait référence aux tableaux 1 à 4 qui représentent la distribution granulométrique de microsphères préparées selon l'invention, la cinétique de libération in vitro de microsphères de diamètre compris entre 160 et 200 µm, et l'activité thérapeutique de celles-ci.

<u>EXEMPLE 1</u> Préparation de microsphères de primaquine diphosphate incorporée dans du polylactide (DL).

On dissout 2,7 g de polylactide (DL) dans 21 $cm^3$ d'acétone sous agitation magnétique pendant une nuit (16% de polymère par rapport au poids d'acétone). On ajoute du monooléate de sorbitane (span® 80) en quantité de 0,83 g (5% par rapport au poids d'acétone). On ajoute ensuite 0,7 g de primaquine diphosphate. Cette quantité représente 25% par rapport au poids de polymère. On procède à la dispersion de cette poudre dans la solution acétonique de polymère, refroidie à 0°C, par l'action d'ultrasons.

Après la dissolution du polymère et la dispersion du principe actif aux ultrasons, on procède immédiatement à l'émulsification. Celle-ci se fait à 0°C sous agitation mécanique (moteur avec tige à 4 pales tournant à 800 tours/minute en versant lentement la suspension acétonique dans 100 $cm^3$ de paraffine liquide contenue dans un bécher de 250 ml plongeant dans de la glace fondante. Ensuite a lieu l'étape d'évaporation du solvant. Le bécher est alors couvert par une plaque en verre percée de deux orifices : l'un permet le passage de la tige d'agitation, l'autre un raccordement à une bonbonne d'azote. L'agitation mécanique est alors réduite à 700 tours/minute jusqu'à la fin de la préparation. C'est en élevant progressivement la température du bain extérieur que le solvant s'avapore pour passer d'une émulsion à une suspension de microparticules, cette opération s'effectuant sous léger courant d'azote en deux phases :

— évaporation lente à 0°C, pendant trois heures puis à 15°C, durant 15 heures ;
— évaporation rapide ; on élève alors la température par pas de 5°C (pendant 15 minutes à 15 et 20°C, pendant 30 minutes à 25, 30 et 35°C et pendant 75 minutes à 40°C.

On procède ensuite à la récupération des microsphères en centrifugeant pendant 10 minutes, puis en filtrant et en lavant à l'heptane qui est un non solvant des deux constituants. Ensuite a lieu le séchage à l'étuve à vide, suivi du tamisage.

Une évaporation rapide de l'acétone à une température de 35°C en travaillant à bécher "ouvert" conduit à la formation de microsphères creuses voire éclatées, c'est pourquoi nous avons réduit la vitesse d'évaporation de l'acétone. On a ainsi obtenu des microparticules sphériques et pleines.

De manière à réduire la taille des microsphères préparées dans le système acétone-paraffine avec les esters de sorbitane, la quantité d'agent tensio-actif doit être comprise entre 1 et 10% par rapport au poids d'acétone et de préférence 5% avec le span® 80.

Des essais ont été réalisés en utilisant différents tensio-actifs à différentes concentrations. Les tensio-actifs utilisés ont été les suivants : tristéarate d'aluminium, et les différents esters de sorbitane, notamment :

— le span® 85 (trioléate de sorbitane HLB = 1,8)
— l'arlacel® 83 (sesquioléate de sorbitane HLB = 3,7)
— le span® 80 (monooléate de sorbitane HLB = 4,3) et
— le span® 60 (monostéarate de sorbitane HLB = 4,7).

Ces esters de sorbitane ont été choisis en fonction de la balance hydrophile-lipophile de leurs molécules (HLB). Un HLB de faible valeur va donc caractériser un produit qui est plus lipophile qu'hydrophile.

Il apparaît que les microsphères de taille moyenne la plus faible sont obtenues avec le span® 80 à 5%.

Toutefois, on obtient des particules de tailles plus réduites avec les autres esters de sorbitane qu'avec le tristéarate d'aluminium.

Les conditions d'émulsification sont également extrêmement critiques. Soumettant le système à une agitation mécanique de 800 tours/minute pendant l'ajout, on réduit considérablement la taille moyenne des microsphères. On arrive ainsi à obtenir une proportion importante de microsphères de taille inférieure à 200 µm (tableau 1). Pour les études in vitro et in vivo, nous utiliserons la fraction granulométrique comprise entre 160 et 200 µm.

Le taux de charge, c'est-à-dire le taux d'incorporation en primaquine dans les microsphères, est proportionnel à la taille des microsphères. Pour des microsphères de taille comprise entre 160 et 200 µm, on obtient des taux de charge en principe actif de l'ordre de 15%.

Dans la présente demande, le "taux de charge" s'entend du rapport T.C. : (poids de principe actif)/(poids de microsphères).

La mise en suspension du phosphate de primaquine dans la solution acétonique de polymère n'est pas aisée, le principe actif s'agglomérant sous forme de grumeaux difficiles à disperser. Il est important d'utiliser le principe actif sous la forme granulométrique la plus fine. Des résultats satisfaisants ont été obtenus par dispersion de la poudre aux ultrasons, à la puissance de 100 watts pendant quelques minutes.

S'agissant des polymères, les essais ont été réalisés avec du polylactide DL de masses moléculaires moyennes en nombre et en poids de l'ordre de 55.000 et 100.000 respectivement (déterminées par chromatographie de perméation sur gel avec des étalons de polystyrène).

TABLEAU 1 : Distribution granulométrique (%) de quatre préparations de microsphères de polylactide (DL) contenant la primaquine diphosphate.

| Echantillon | Diamètre des microparticules (µm) | | | | | |
|---|---|---|---|---|---|---|
| | 70-100 | 100-160 | 160-200 | 200-250 | 250-315 | > 315 |
| ST 11 | 0,1 | 5,1 | 17,9 | 50,0 | 23,6 | 3,3 |
| ST 12 | 3,3 | 45,1 | 39,8 | 10,0 | 1,4 | 0,2 |
| ST 71 | 0,3 | 7,5 | 24,0 | 47,8 | 18,6 | 1,8 |
| ST 81 | 1,2 | 43,1 | 41,8 | 11,0 | 2,1 | 0,7 |

EXEMPLE 2 : Cinétique de libération in vitro de microsphères préparées selon la méthode décrite à l'exemple 1.

Pour les divers échantillons repris au tableau 1, nous avons isolé par tamisage mécanique la fraction granulométrique comprise entre 160 et 200 µm, déterminé son taux de charge (dosage colorimétrique de la primaquine extraite en phase aqueuse après dissolution des microsphères au dichlorométhane) et suivi la libération du principe actif pendant 16 jours. En pratique, on utilise 10-20 mg de microsphères pour 50 ml de tampon phosphate isotonique à pH 7,4. Cette suspension est agitée dans un bain oscillant à 37°C. Après 1 heure (libération massive ou "burst effect"), et quotidiennement, on mesure l'absorbance du milieu à 259 nm, après décantation des microsphères. Les résultats repris au tableau 2 montrent que ces microsphères ne présentent pas ou peu (4-5%), de libération massive ou "burst effect", et se caractérisent par une cinétique d'ordre zéro pendant une dizaine de jours, avec une vitesse de libération journalière comprise entre 7,7 et 11,1%.

TABLEAU 2 : Taux de charge et cinétique de libération in vitro de la fraction granulométrique 160-200 µm.

| Echantillon | Taux de charge (%) | Quantité libérée après 1 heure (%) | Vitesse de libération moyenne (%/jour) |
|---|---|---|---|
| ST 11 | 15,0 | 0 | 8,0 |
| ST 12 | 14,5 | 0 | 11,1 |
| ST 71 | 16,1 | 4,2 | 7,7 |
| ST 81 | 17,9 | 4,8 | 8,6 |

EXEMPLE 3 : Activité prophylactique préventive de la primaquine diphosphate (Pq) incorporée dans des microsphères de polylactide (DL) de taille comprise entre 160 et 200 µm.

Des expériences ont été faites avec la primaquine incorporée dans un polymère biodégradable, la polylactide (DL) avec un poids moléculaire de l'ordre de 55.000, sous la forme galénique de microsphères.

L'activité préventive de la primaquine ainsi incorporée a été déterminée dans le modèle expérimental de la malaria murine (Plasmodium berghei, souche ANKA, souris swiss OF1 femelles). Les microsphères, d' une taille granulométrique comprise entre 160 et 200 µm ont été administrées par voie intramusculaire, 3 à 17 jours avant l'infection des souris avec les sporozoïtes de P. berghei.

Comme décrit dans l'exemple 2, les préparations de microsphères de poly (DL) lactide contenant la primaquine, ont un burst effect réduit et une libération régulière comprise entre 9 et 14 jours. Le schéma thérapeutique (doses injectées, périodes de couverture) a été sélectionné sur base de la libération moyenne journalière caractéristique de chacune des préparations ST11 (8% de Pq), ST12 (11,1% de Pq) et ST71 (7,7% de Pq).

Les résultats obtenus sont présentés dans le tableau 3. Il ressort de cette étude que :

1. La concentration journalière en Pq nécessaire pour obtenir une protection complète des animaux infectés est d'environ 50 mg Pq/kg pour autant que la libération chez l'animal soit similaire à la libération in vitro.

2. La période de couverture optimale est, dans le modèle de la malaria murine, de 14 jours.

Ainsi, pour l'échantillon ST11, une protection partielle est obtenue à la dose de 450 mg Pq/kg soit 36 mg/kg/jour et la protection complète des animaux infectés à la dose de 625 mg Pq/kg soit 50 mg Pq/kg/jour. Dans le cas de l'échantillon ST71, la dose de 700 mg Pq/kg (50 mg Pq/kg/jour) est totalement curative à 14 jours mais n'est plus que partiellement active à 17 jours.

TABLEAU 3 : Activité prophylactique préventive de la primaquine diphosphate incorporée dans des microsphères de polylactide (DL)

| Echantillon | Taux de charge (%) | Dose[a] (mg/kg) | Traitement[b] (jours) | LTS/N | LTS[c] (%) |
|---|---|---|---|---|---|
| ST11 | 15 | 450 | -7 | 3/5 | 60 |
| | | 625 | -7 | 5/5 | 100 |
| ST12 | 14,5 | 450 | -3 | 5/5 | 100 |
| | | 450 | -7 | 5/5 | 100 |
| ST71 | 16,1 | 700 | -10 | 5/5 | 100 |
| | | 700 | -14 | 5/5 | 100 |
| | | 700 | -17 | 3/5 | 60 |

a : Les microsphères de taille granulométrique comprise entre 160 et 200 µm, sont injectées en solution de miglyol 812 (viscosifié par 4 % de Thixcin R) par voie intramusculaire. Les concentrations sont exprimées en mg de primaquine diphosphate/kg.

b : Les animaux traités sont infectés avec des sporozoïtes de P. berghei, 3 à 17 jours après le traitement.

c : Activité prophylactique préventive (%) = le nombre d'animaux protégés sur le nombre d'animaux traités et infectés (exprimé en %).

EXEMPLE 4 : Préparation de microsphères de L-glutamyl-primaquine incorporée dans du polylactide (DL).

A) SYNTHESE DES DERIVES AMINOACYLES DE LA PRIMAQUINE

Les dérivés aminoacylés de la primaquine sont obtenus en deux étapes au départ de la primaquine diphosphate et de l'acide aminé dont la fonction aminée est protégée par un groupe N-terbutyloxycarbonyle (N-TBoc). La première étape comprend la réaction de la primaquine base avec l'ester N-hydroxysuccinimide du dérivé N-TBoc de l'acide aminé.

Le produit brut ainsi obtenu est alors purifié par chromatographie sur silicagel. On isole avec de bons rendements (± 70%) le produit obtenu sous forme d'un solide ± cristallin assez hygroscopique.

Le groupe TBoc est ensuite clivé en présence d'acide trifluoroacétique. Le dérivé aminoacyle de la primaquine est obtenu sous forme trifluoroacétate (probablement un sel ditrifluoroacétate).

Dans le cas où une purification ultérieure est nécessaire, on utilise une colonne de chromatographie à phase inverse et le produit est élué par le mélange acétonitrile/$H_2O$.

Le dérivé aminoacyle de la primaquine sous sa forme base libre peut être obtenu en neutralisant la solution aqueuse contenant le sel de trifluoroacétate d'aminoacyle-Pq par $NH_4OH$ jusqu'à pH ± 8 suivi d'une extraction

vigoureuse par le dichlorométhane. On obtient, après évaporation du $CH_2Cl_2$, une huile verdâtre trés difficilement manipulable qui peut être purifiée ultérieurement par chromatographie sur colonne de silicagel élué avec un mélange $CH_2Cl_2$— Et OH-$NH_4OH$ (120-20-1).

Dans la méthode de purification des dérivés aminoacyles de la primaquine sous leur forme trifluoroacétate utilisant la technique de chromatographie sur colonne phase inverse, la colonne utilisée est une colonne Merck lobar 8. Le support est une silice greffée par des résidus en $C_8$. Afin d'éviter un processus d'oxydation des dérivés sur la colonne, les solvants d'élution sont soigneusement dégazés. Pour la même raison, on peut envisager d'autres types de silice greffée comme phase stationnaire, notamment complètement déminéralisés. Le produit brut dissous en milieu acide aqueux est adsorbé en tête de colonne ; on procède ensuite à une étape de désalage ($H_2O$-$H^+$) avant d'éluer le produit en utilisant un gradient de $CH_3CN$ dans $H_2O/H^+$. L'éluant est fractionné. Les fractions contenant le produit (fractions colorées jaune orange) sont analysées par HPLC. Celles contenant le produit avec une pureté supérieure à 98% sont rassemblées et lyophilisées. On obtient une poudre jaune orange vive. Le contre anion trifluoroacétate est alors échangé en chlorhydrate en additionnant à une solution aqueuse du sel de trifluoroacétate une quantité d'HCl aqueuse correspondant approximativement à deux équivalents d'acide calculé par rapport au poids de sel de trifluoroacétate à échanger. La solution obtenue (pH 2,8-3) est lyophilisée à l'abri de la lumière (2 lyophilisations). Le produit obtenu se présente sous forme d'une poudre orange vive très hygroscopique. Même stocké sous atmosphère inerte (argon), le produit se dégrade assez rapidement au cours du temps. Il présente également une stabilité limitée en solution : milieu physiologique ou solution aqueuse à 0° et à l'abri de la lumière.

Le noyau quinoléique est une cible particulièrement sensible pour toute réaction d'oxydation. Celle-ci explique partiellement l'instabilité observée. Dans le but d'inhiber ces réactions d'oxydation, nous avons traité la solution aqueuse contenant le sel de trifluoroacétate par du bisulfite de sodium (0,1%).

La proportion relative $CH_3CN$-$H_2O/H^+$ varie suivant la nature et le nombre d'acides aminés greffés sur la primaquine.

Synthèse de la N-1-L-glutamyl, N-4-(méthoxy-6-quinolinyl-8) pentadiamine-1,4

A 5,9 mmoles de N-tBoc-glutamique, dissous dans 10 ml de diglyme, maintenu à 0°C, on ajoute 0,61 g (5,3 mmoles) de la N-hydroxysuccinimide et 1,09 g (5,3 mmoles) de dicyclohexylcarbodiimide. Après 4 heures de réaction, on ajoute 1,37 g (5, 3 mmoles) de primaquine base. Cette dernière est libérée de la primaquine diphosphate par l'action d'une solution d'ammoniaque 25%. Après 17 heures, la dicyclohexylurée est filtrée et le solvant chassé sous vide. L'huile brune obtenue est dissoute dans le $CH_2Cl_2$ et lavée à l'eau. La phase organique est séchée sur sulfate de sodium, filtrée et évaporée. Le produit est purifié sur gel de silice en utilisant comme éluant un mélange dichlorométhane-méthanol. Les fractions pures sont rassemblées et l'éluant évaporé conduisant à 1,5 g d'un produit plus ou moins cristallin, assez hygroscopique.

Le composé obtenu précédemment est dissous dans 15 ml d'un mélange 1 : 1 de dichlorométhane et d'acide trifluoroacétique. Le mélange est agité 30 minutes à température ambiante. Les solvants sont évaporés sous vide et le résidu est repris dans de l'eau et lavé plusieurs fois au diisopropyléther.

Si nécessaire, le produit est purifié par chromatographie sur phase inverse (Merck lobar 8) en utilisant comme éluant un mélange de 25% d'acétonitrile dans de l'eau contenant 1% d'acide trifluoroacétique. Les fractions pures (HPLC) sont rassemblées, l'acétonitrile chassé sous vide. Après 2 cycles de lyophilisation, on obtient 1,6 g (80%) d'une poudre orange vive hygroscopique.

B) MICROSPHERES :

Les microsphères ont été préparées selon le mode opératoire présenté à l'exemple 1. La distribution granulométrique de trois préparations est présentée au tableau 4. Comme dans le cas de la primaquine diphosphate, on obtient une proportion importante de microsphères injectables.

TABLEAU 4 : Distribution granulométrique (%) de trois préparations de microsphères de polylactide (DL) contenant la L-glutamyl primaquine.

| Echantillon | Diamètre des microparticules (µm) | | | | | |
|---|---|---|---|---|---|---|
| | 70-100 | 100-160 | 160-200 | 200-250 | 250-315 | >315 |
| PH 52A | 1,1 | 14,8 | 31,9 | 43,2 | 8,4 | 0,5 |
| PH 52B | 1,3 | 18,8 | 19,8 | 20,6 | 28,9 | 10,6 |
| PH 52C | 0,5 | 5,7 | 16,4 | 49,6 | 26,4 | 1,3 |

**Revendications**

**Revendications pour les Etats Contractants AT BE CH DE FR GB IT LI LU NL SE**

1. Microparticules consistant en des microsphères de 160 à 200 µm constituées d'une matrice de polymère polylactide, de préférence sous forme (DL) au sein de laquelle est distribué un principe actif qui est la primaquine, un de ses dérivés de formule :

$$PQ - X$$

dans laquelle,

PQ représente la primaquine,

X représente un amino-acide ou un peptide de 2 à 4 amino-acides, la liaison PQ-X étant une liaison covalente peptidique entre le groupement amine libre de PQ et le groupement carboxylique de X,

ou les conjugués de PQ ou PQ-X avec un vecteur hépatotrope, ou un de leurs sels pharmaceutiquement acceptables, le poids moléculaire du polymère étant compris entre 50000 et 100000, le rapport en poids principe actif/polymère étant de 15 à 25%.

2. Compositon pharmaceutique, caractérisée en ce qu'elle contient des microparticules selon l'une des revendications précédentes.

3. Composition selon la revendication 2, caractérisée en ce qu'elle est injectable.

4. Procédé de préparation de microparticules, selon l'une des revendications précédentes, caractérisé en ce que :

- le polymère est dissous dans un solvant volatil,
- à cette solution, on ajoute le principe actif et éventuellement une substance réglant la taille des microparticules,
- à l'issue de l'évaporation, les microparticules sont récupérées par centrifugation et filtration.

5. Procédé selon la revendication 4, caractérisé en qu'on incorpore la primaquine ou un de ses dérivés ou conjugués, ou un de leurs sels pharmaceutiquement acceptables dans du polylactide, le solvant étant l'acétone et le milieu dispersant étant une huile minérale, telle que la paraffine, la substance réglant la taille des particules étant un agent tensio-actif non ionique.

6. Procédé selon la revendication 5, caractérisé en ce que le polylactide est sous forme (DL).

7. Procédé selon l'une des revendications 4 à 6, caractérisé en ce que le rapport en poids polymère/solvant est de 10 à 30%, de préférence 15 à 20% et le rapport en poids principe actif/polymère est de 1 à 50%, de préférence 15 à 25%.

8. Procédé selon l'une des revendications 4 à 7, caractérisé en ce que la dispersion du principe actif dans l'acétone se fait par l'utilisation des ultrasons.

9. Procédé selon l'une des revendications 5 à 8, caractérisé en ce qu'on utilise comme détergent non ionique un ester de sorbitane, de préférence dans une concentration comprise entre 1 et 10%.

10. Procédé selon l'une des revendications 5 à 9, caractérisé en ce que l'ester de sorbitane est le monooléate de sorbitane utilisé à raison de 5% en poids par rapport à l'acétone.

**Revendications pour les Etats Contractants ES et GR**

1. Procédé de préparation de microparticules consistant en des microsphères de 160 à 200 µm constituées d'une matrice de polymère polylactide, de préférence sous forme (DL) au sein de laquelle on distribue un principe actif qui est la primaquine, un de ses dérivés de formule :

PQ – X

dans laquelle,

PQ représente la primaquine,

X représente un amino-acide ou un peptide de 2 à 4 amino-acides, la liaison PQ-X étant une liaison covalente peptidique entre le groupement amine libre de PQ et le groupement carboxylique de X,

ou les conjugués de PQ ou PQ-X avec un vecteur hépatotrope, ou un de leurs sels pharmaceutiquement acceptables, le poids moléculaire du polymère étant compris entre 50000 et 100000, le rapport en poids principe actif/polymère étant de 15 à 25%.

2. Procédé de préparation de microparticules, selon la revendication 1, caractérisé en ce que :

- le polymère est dissous dans un solvant volatil,
- à cette solution, on ajoute le principe actif et éventuellement une substance réglant la taille des microparticules,
- à l'issue de l'évaporation, les microparticules sont récupérées par centrifugation et filtration.

3. Procédé selon la revendication 2, caractérisé en ce qu'on incorpore la primaquine ou un de ses dérivés ou conjugués, ou un de leurs sels pharmaceutiquement acceptables dans du polylactide, le solvant étant l'acétone et le milieu dispersant étant une huile minérale, telle que la paraffine, la substance réglant la taille des particules étant un agent tensio-actif non ionique.

4. Procédé selon la revendication 3, caractérisé en ce que le polylactide est sous forme (DL).

5. Procédé selon l'une des revendications 2 à 4, caractérisé en ce que le rapport en poids polymère/solvant est de 10 à 30%, de préférence 15 à 20%, et le rapport en poids principe actif/polymère est de 1 à 50%, de préférence 15 à 25%.

6. Procédé selon l'une des revendications 2 à 5, caractérisé en ce que la dispersion du principe actif dans l'acétone se fait par l'utilisation des ultrasons.

7. Procédé selon l'une des revendications 3 à 6, caractérisé en ce qu'on utilise comme détergent non ionique un ester de sorbitane, de préférence dans une concentration comprise entre 1 et 10%.

8. Procédé selon l'une des revendications 3 à 7, caractérisé en ce que l'ester de sorbitane est le monooléate de sorbitane utilisé à raison de 5% en poids par rapport à l'acétone.

**Claims**

**Claims for the Contracting States AT, BE, CH, DE, FR, GB, IT, LI, LU, NL, SE**

1. Microparticles consisting of microspheres of 160 to 200 µm constituted by a polylactide polymer matrix, preferably in (DL) form, within which is distributed an active principle which is primaquine, one of its derivatives of the formula :

PQ – X

in which,

PQ represents primaquine,

X represents an amino acid or a peptide of 2 to 4 amino acids, the linkage PQ-X being a peptide covalent linkage between the free amine group of PQ and the carboxylic groupe of X,

or conjugates of PQ or PQ-X with a hepatotropic vector, or one of their pharmaceutically acceptable salts, the molecular weight of the polymer being comprised between 50,000 and 100,000, and the ratio by weight active principle/polymer being from 15 to 25%.

2. Pharmaceutical composition, which contains microparticles according to claim 1.

3. Composition according to claim 2, said composition being injectable.

4. Process for the preparation of microparticles, according to any of precedent claims, said process comprising :

— dissolving the polymer in a volatile solvent,

— adding to this solution, the active principle and possibly a substance regulating the size of the microparticles,

— after evaporation, recovering the microparticles by centrifugation and filtration.

5. Process according to claim 4, wherein the primaquine or one of its derivatives or conjugates, or one of their pharmaceutically acceptable salts is incorporated in polylactide, the solvent being acetone and the dispersing medium being a mineral oil, such as paraffin, the substance regulating the size of the particles being a non-ionic surface-active agent.

6. Process according to claim 5, wherein the polylactide is in (DL) form.

7. Process according to one of claims 4 to 6, wherein the ratio by weight of polymer/solvent is from 10 to 30%, preferably 15 to 20%, and the ratio by weight of active principle/polymer is from 1 to 50%, preferably 15 to 25%.

8. Process according to one of claims 4 to 7, wherein the dispersion of the active principle in the acetone is done by the use of ultrasound.

9. Process according to one of claims 5 to 8, comprising using as non-ionic detergent, a sorbitan ester preferably in a concentration comprised between 1 and 10%.

10. Process according to one of claims 5 to 9, wherein the sorbitan ester is sorbitan monooleate used in the proportion of 5% by weight with respect to the acetone.

**Claims for the Contracting States ES, GR**

1. Process of preparation of microparticles consisting of microspheres of 160 to 200 μm constituted by a polylactide polymer matrix, preferably in (DL) form, within which one distributes an active principle which is primaquine, one of its derivatives of the formula :

$$PQ - X$$

in which,

PQ represents primaquine,

X represents an amino acid or a peptide of 2 to 4 amino acids, the linkage PQ-X being a peptide covalent linkage between the free amino group of PQ and the carboxylic group of X,

or conjugates of PQ or PQ-X with a hepatotropic vector, or one of their pharmaceutically acceptable salts, the molecular weight of the polymer being comprised between 50,000 and 100,000, and the ratio by weight active principle/polymer being from 15 to 25%.

2. Process for the preparation of microparticles, according to claim 1, said process comprising :

— dissolving the polymer in a volatile solvent,

— adding to this solution, the active principle and possibly a substance regulating the size of the microparticles,

— after evaporation, recovering the microparticles by centrifugation and filtration.

3. Process according to claim 2, wherein the primaquine or one of its derivatives or conjugates, or one of their pharmaceutically acceptable salts is incorporated in polylactide, the solvent being acetone and the dispersing medium being a mineral oil, such as paraffin, the substance regulating the size of the particles being a non-ionic surface-active agent.

4. Process according to claim 3, wherein the polylactide is in (DL) form.

5. Process according to one of claims 2 to 4, wherein the ratio by weight of polymer/solvent is from 10 to 30%, preferably 15 to 20%, and the ratio by weight of active principle/polymer is from 1 to 50%, preferably 15 to 25%.

6. Process according to one of claims 2 to 5, wherein the dispersion of the active principle in the acetone is done by the use of ultrasound.

7. Process according to one of claims 3 to 6, comprising using as non-ionic detergent, a sorbitan ester preferably in a concentration comprised between 1 and 10%.

8. Process according to one of claims 3 to 7, wherein the sorbitan ester is sorbitan monooleate used in the proportion of 5% by weight with respect to the acetone.

## Patentansprüche

### Patentansprüche für den Vertragstaaten AT, BE, CH, DE, FR, GB, IT, LI, LU, NL, SE

1. Mikroteilchen, bestehend aus Mikrokugeln von 160 bis 200 µm, gebildet aus einer Polylactid-Polymer-Matrix, vorzugsweise in der DL-Form, in der ein wirksames Prinzip verteilt ist, bei dem es sich um Primaquin, eines seiner Derivate der Formel

PQ – X

worin

PQ das Primaquin darstellt,

X eine Aminosäure oder ein Peptid aus 2 bis 4 Aminosäuren darstellt, wobei die Bindung PQ-X eine kovalente Peptidbindung zwischen der freien Amingruppe von PQ und der Carboxylgruppe von X ist,

oder die Konjugate von PQ oder PQ-X mit einem hepatotropen Vector, oder eines ihrer pharmazeutisch brauchbaren Salze handelt, wobei das Molekulargewicht des Polymeren bei 50000 bis 100000 liegt und das Gewichtsverhältnis von wirksamem Prinzip/Polymer 15 bis 25% beträgt.

2. Pharmazeutische Zusammensetzung, dadurch gekennzeichnet, daß sie Mikroteilchen gemäß einem der vorhergehenden Ansprüche enthält.

3. Zusammensetzung nach Anspruch 2, dadurch gekennzeichnet, daß sie injizierbar ist.

4. Verfahren zur Herstellung von Mikroteilchen nach einem der vorhergehenden Ansprüche, dadurch gekennzeichnet, daß :

- das Polymere in einem flüchtigen Lösungsmittel gelöst wird,
- man zu dieser Lösung das wirksame Prinzip und gegebenenfalls eine die Größe der Mikroteilchen regelnde Substanz fügt,
- nach der Verdampfung die Mikroteilchen durch Zentrifugieren und Filtrieren gewonnen werden.

5. Verfahren nach Anspruch 4, dadurch gekennzeichnet, daß man das Primaquin oder eines seiner Derivate oder Konjugate oder eines ihrer pharmazeutisch brauchbaren Salze in Polylactid einarbeitet, wobei das Lösungsmittel Aceton und das Dispergiermedium ein Mineralöl, wie Paraffin ist und die die Teilchengröße regelnde Substanz ein nichtionisches oberflächenaktives Mittel ist.

6. Verfahren nach Anspruch 5, dadurch gekennzeichnet, daß das Polylactid in der DL-Form vorliegt.

7. Verfahren nach einem der Ansprüche 4 bis 6, dadurch gekennzeichnet, daß das Gewichtsverhältnis Polymer/Lösungsmittel 10 bis 30%, vorzugsweise 15 bis 20%, und das Gewichtsverhältnis wirksames Prinzip/Polymer 1 bis 50%, vorzugsweise 15 bis 25%, beträgt.

8. Verfahren nach einem der Ansprüche 4 bis 7, dadurch gekennzeichnet, daß das Dispergieren des wirksamen Prinzips in Aceton unter Anwendung von Ultraschall erfolgt.

9. Verfahren nach einem der Ansprüche 5 bis 8, dadurch gekennzeichnet, daß man als nicht-ionisches Detergens einen Sorbitanester, vorzugsweise in einer Konzentration von 1 bis 10%, verwendet.

10. Verfahren nach einem der Ansprüche 5 bis 9, dadurch gekennzeichnet, daß der Sorbitanester Sorbitanmonooleat, verwendet in einer Menge von 5 Gew.%, bezogen auf das Aceton, ist.

### Patentansprüche für Vertragstaaten ES, GR

1. Verfahren zur Herstellung von Mikroteilchen, die aus Mikrokugeln von 160 bis 200 µm bestehen, gebildet aus einer Polylactid-Polymer-Matrix, vorzugsweise in der DL-Form, in der man ein wirksames Prinzip verteilt, bei dem es sich um Primaquin, eines seiner Derivate der Formel

PQ – X

worin

PQ das Primaquin darstellt,

X eine Aminosäure oder ein Peptid aus 2 bis 4 Aminosäuren darstellt, wobei die Bindung PQ-X eine kovalente Piptidbindung zwischen der freien Amingruppe von PQ und der Carboxylgruppe von X ist,

oder die Konjugate von PQ oder PQ-X mit einem hepatotropen Vector, oder deren pharmazeutisch brauchbare Salze handelt, wobei das Molekulargewicht des Polymeren bei 50000 bis 100000 liegt und das Gewichtsverhältnis wirksames Prinzip/Polymer 15 bis 25% beträgt.

2. Verfahren zur Herstellung von Mikroteilchen nach Anspruch 1, dadurch gekennzeichnet, daß

- das Polymere in einem flüchtigen Lösungsmittel gelöst wird,

• man zu dieser Lösung das wirksame Prinzip und gegebenenfalls eine die Größe der Mikroteilchen regelnde Substanz fügt,

• nach der Verdampfung die Mikroteilchen durch Zentrifugieren und Filtrieren gewonnen werden.

3. Verfahren nach Anspruch 2, dadurch gekennzeichnet, daß man das Primaquin oder eines seiner Derivate oder Konjugate oder eines ihrer pharmazeutisch brauchbaren Salze in Polylactid einarbeitet, wobei das Lösungsmittel Aceton und das Dispergiermedium ein Mineralöl, wie Paraffin ist und die die Teilchengröße regelnde Substanz ein nichtionisches oberflächenaktives Mittel ist.

4. Verfahren nach Anspruch 3, dadurch gekennzeichnet, daß das Polylactid in der DL-Form vorliegt.

5. Verfahren nach einem der Ansprüche 2 bis 4, dadurch gekennzeichnet, daß das Gewichtsverhältnis Polymer/Lösungsmittel 10 bis 30%, vorzugsweise 15 bis 20%, und das Gewichtsverhältnis wirksames Prinzip/Polymer 1 bis 50%, vorzugsweise 15 bis 25%, beträgt.

6. Verfahren nach einem der Ansprüche 2 bis 5, dadurch gekennzeichnet, daß das Dispergieren des wirksamen Prinzips in Aceton unter Anwendung von Ultraschall erfolgt.

7. Verfahren nach einem der Ansprüche 3 bis 6, dadurch gekennzeichnet, daß man als nicht-ionisches Detergens einen Sorbitanester, vorzugsweise in einer Konzentration von 1 bis 10%, verwendet.

8. Verfahren nach einem der Ansprüche 3 bis 7, dadurch gekennzeichnet, daß der Sorbitanester Sorbitanmonooleat, verwendet in einer Menge von 5 Gew.%, bezogen auf das Aceton, ist.